(19)

Europäisches
Patentamt

European
Patent Office

Office européen
des brevets

(11) **EP 4 166 075 A1**

(12) **EUROPEAN PATENT APPLICATION**
published in accordance with Art. 153(4) EPC

(43) Date of publication:
**19.04.2023 Bulletin 2023/16**

(21) Application number: **21841228.6**

(22) Date of filing: **15.07.2021**

(51) International Patent Classification (IPC):
***A61B 5/053*** (2021.01)　　***A61B 5/00*** (2006.01)

(52) Cooperative Patent Classification (CPC):
**A61B 5/00; A61B 5/053**

(86) International application number:
**PCT/KR2021/009122**

(87) International publication number:
**WO 2022/015081 (20.01.2022 Gazette 2022/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.07.2020　KR 20200088362
12.07.2021　KR 20210091081**

(71) Applicant: **Samsung Electronics Co., Ltd.
Gyeonggi-do 16677 (KR)**

(72) Inventors:
• **PARK, Jaehyuck**
　**Suwon-si Gyeonggi-do 16677 (KR)**
• **KWON, Hyoujoo**
　**Suwon-si Gyeonggi-do 16677 (KR)**
• **LEE, Wonseok**
　**Suwon-si Gyeonggi-do 16677 (KR)**
• **CHANG, Namseok**
　**Suwon-si Gyeonggi-do 16677 (KR)**

(74) Representative: **Gulde & Partner
Patent- und Rechtsanwaltskanzlei mbB
Wallstraße 58/59
10179 Berlin (DE)**

(54) **WEARABLE DEVICE AND METHOD FOR MEASURING HUMAN BODY IMPEDANCE**

(57)　An embodiment of the present disclosure discloses a device comprising a housing and a biometric sensor, the biometric sensor is configured to sense whether a first part of a human body is in contact with a first electrode and a second electrode, detect whether a second part of the human body is in contact with a third electrode and a fourth electrode, obtain phase information of the impedance of the human body by using the first, second, third and fourth electrodes, determine whether the acquired phase information of the impedance is within a designated range, and provide a guide for a measurement method when the acquired phase information of the impedance deviates from the designated range. Various other embodiments identified through the specification are possible.

FIG.1

EP 4 166 075 A1

**Description**

[Technical Field]

Various embodiments of the disclosure relate to a wearable device and a method for measuring biometric information, and to an electronic device and a method for detecting a case in which both hands of a user come into contact with each other when measuring biometric information.

[Background Art]

The disclosure relates to a body component measurement device using a bioelectrical impedance and, specifically, to a method and a device for detecting a case in which both hands of a user come into contact with each other.

The main components that make up a body include four principal components including: water, protein, body fat, and minerals. The ratio between these components in the body weight varies by gender and individual, but is approximately 55:20:20:5. These four components may be identified from the total body water. The protein and the total body water are main components for forming muscle and have a mutually proportional relationship, and thus an amount of protein and inorganic mass may be acquired from an amount of the total body water and an amount of body fat may be acquired by subtracting the amount of the total body water, protein, and inorganic mass from a body weight. A most frequently used method for measuring body fat may be a bioelectrical impedance analysis (BIA) and an underwater body density method, a computed tomography, and a subcutaneous fat thickness measurement method, and the like may be used in addition thereto.

The bioelectrical impedance analysis is a method of measuring body fat based on the fact that an amount of the total body water and a body's electrical resistance are in inverse proportion.

The bioelectrical impedance analysis has an advantage that measurement is simple, fast, and non-invasive. When a weak alternating current electrical signal is applied to a human body, the electricity flows along the total body water having high conductivity. The width of a path through which the electricity flows is determined according to an amount of moisture, and a measurement value according thereto is a bio-impedance. A method of calculating a body component from the bio-impedance includes applying a fine alternating current through the human body in a frequency band of 50 kHz. When the current flows, a human body resistance is measured and used to calculate a total body water amount. A protein amount and an inorganic material amount are calculated from the total body water amount and body fat is calculated by using the protein amount, the inorganic material amount, and a body weight.

[Disclosure of Invention]

[Technical Problem]

A conventional wearable device for measuring a body component may require some electrodes to be contact with one wrist of one arm and another electrode to be contact with the other arm or a finger so as to accurately measure human body impedance. However, accurate measurement of human body impedance may be difficult when both arms are in contact with each other due to user's negligence.

In addition, from the point of view of a wearable device for easy measurement in everyday life, rather than a device for measurement under limited and strict conditions such as measurement in hospitals, it may be difficult to demand a user a strict posture condition.

Therefore, various embodiments of the disclosure describe a method for detecting a case in which both arms are in contact to each other so as to inform a user that measurement is not performed properly and guide remeasurement.

[Technical Solution]

An electronic device according to an embodiment may include a housing and a biometric sensor, wherein the biometric sensor is configured to sense whether a first part of a human body is in contact with a first electrode and a second electrode, detect whether a second part of the human body is in contact with a third electrode and a fourth electrode, acquire impedance phase information of the human body by using the first, second, third and fourth electrodes, determine whether the acquired impedance phase information is within a designated range, and provide a guide for a measurement method in case that the acquired phase information of the impedance deviates from the designated range.

An operation method of an electronic device according to an embodiment may include an operation of sensing, through a biometric sensor, whether a first part of a human body is in contact with a first electrode and a second electrode, an operation of detecting, through the biometric sensor, whether a second part of the human body is in contact with a third electrode and a fourth electrode, an operation of acquiring impedance phase information of the human body by using the first, second, third, and fourth electrodes, an operation of determining whether the acquired impedance phase information is within a designated range, and an operation of providing a guide for a measurement method in case that the acquired impedance phase information deviates from the designated range.

An electronic device according to an embodiment may include a housing including a first surface configured to come into contact with a first portion of a human body of a user when worn, a second surface configured to not come into contact with the first portion of the human body in case that the first surface comes into contact with the first portion, and a lateral surface configured to surround at least a portion of a space between the first surface and the second surface, a memory, a biometric circuit including a first electrode and a second electrode exposed through the first surface and a third electrode and a fourth electrode exposed through at least one of the second surface and the lateral surface, and at least one processor electrically connected to the biometric circuit and the memory, wherein the at least one processor is configured to measure a parasitic impedance value through the biometric sensor, store the measured parasitic impedance value in the memory, detect, through the biometric circuit, that the first portion of the human body comes into contact with the first electrode and the second electrode, detect, through the biometric circuit, that a second portion of the human body comes into contact with the third electrode and the fourth electrode, apply a current to the human body through the second electrode and the third electrode via the biometric circuit, measure, through the biometric circuit, a voltage across both ends of the first electrode and the fourth electrode in response to the application of the current, acquire impedance phase information of the human body based on the applied current, the measured voltage, and the parasitic impedance value, and provide notification related to biometric data measurement in case that the acquired impedance phase information deviated from a predetermined range.

[Advantageous Effects of Invention]

The electronic device and the method according to various embodiments of the disclosure may provide users with biometric information more accurately and quickly by promptly detecting a situation where body component measurement is not performed properly and guiding a user to measure correctly again.

[Brief Description of Drawings]

FIG. 1 is a view illustrating mounting of a wearable device on a portion of a human body according to an embodiment.
FIG. 2A is a perspective view illustrating a wearable device according to an embodiment.
FIG. 2B is a perspective view illustrating a wearable device according to an embodiment.
FIG. 2C is an exploded view illustrating a lower surface of a wearable device according to an embodiment.
FIG. 3 is a block diagram illustrating a wearable device according to an embodiment.
FIG. 4 is a view illustrating a location of an electrode in a wearable device according to an embodiment.
FIG. 5 is a view illustrating locations of an electrode and a user body coming into contact with the electrode in a wearable device according to an embodiment.
FIG. 6A is a view illustrating a human body closed loop formed in case that a user body comes into contact with an electrode connected to a biometric sensor in a wearable device according to an embodiment.
FIG. 6B is a view illustrating, on a human body, a human body closed loop formed in case that a user body comes into contact with an electrode connected to a biometric sensor in a wearable device according to an embodiment.
FIG. 6C is a view illustrating acquisition of biometric data considering of a parasitic component existing in a biometric contact circuit in a wearable device according to an embodiment.
FIG. 7 is a flowchart illustrating a content for providing a notification related to biometric data measurement to a user according to acquired biometric data in a wearable device according to an embodiment.
FIG. 8 is a flowchart for selecting a control method of a processor according to whether acquired biometric data is within a predetermined range in a wearable device according to an embodiment.
FIG. 9A to FIG. 9E are views illustrating UIs displayed on a display in a wearable device according to various embodiments.
FIG. 10 illustrates a graph indicating a method for determining whether acquired biometric data is within a predetermined range in a wearable device according to an embodiment.
FIG. 11A is a view illustrating a human body closed loop formed in case that an acquired phase information falls within a predetermined range in a wearable device according to an embodiment.
FIG. 11B is a view illustrating a human body closed loop formed in case that both hands of a user are into contact in a wearable device according to an embodiment.
FIG. 11C is a view illustrating a human body closed loop formed in case that an error occurs in an electrode in a wearable device according to an embodiment.
FIG. 12 is a block diagram illustrating an electronic device in a network environment according to an embodiment.

[Best Mode for Carrying out the Invention]

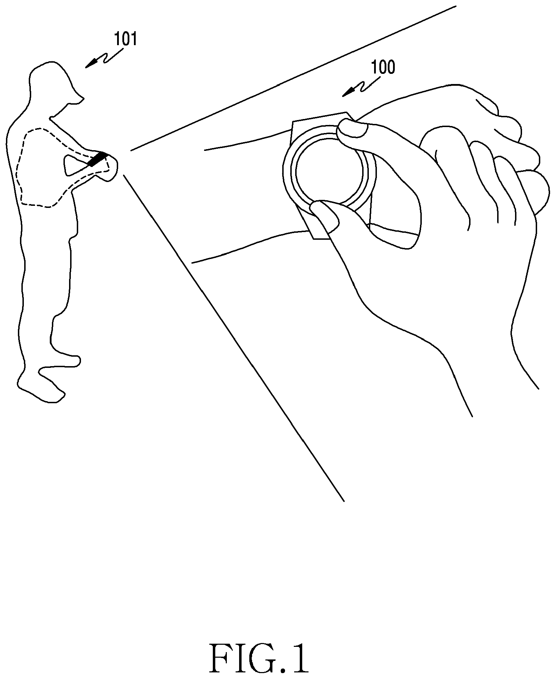

FIG. 1 is a view illustrating mounting of a wearable device on a portion of a human body according to an embodiment.
The wearable device 100 in FIG. 1 according to an embodiment may be a smart watch as shown in the drawing. However, without limitation thereto, the wearable device 100 may include various types of wearable devices attachable to a body of a user 101 to be used.

According to an embodiment, the wearable device 100 may include a strap to be attached to the body of the user 101 by allowing the strap to be wound around a wrist of the user 101. However, without limitation thereto, the wearable device 100 may be attached to various body parts of the user 101 according to a shape, a size, or the like of the wearable device 100. For example, the wearable device 100 may be attached to a hand including a back of the hand, a finger, a fingernail, a fingertip, and the like, as well.

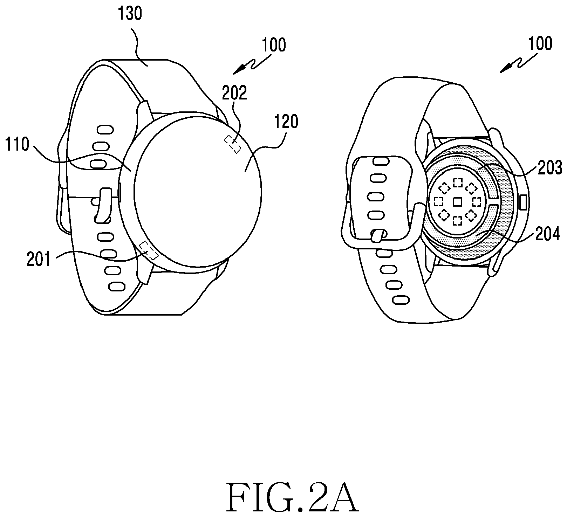

FIG. 2A is a perspective view illustrating a wearable device according to an embodiment.

Referring to FIG. 2A, the wearable device 100 may include a housing 110, an electrode 201, 202, 203, or 204, a display 120, and a strap 130. According to an embodiment, the wearable device 100 may omit at least one component or additionally include other components.

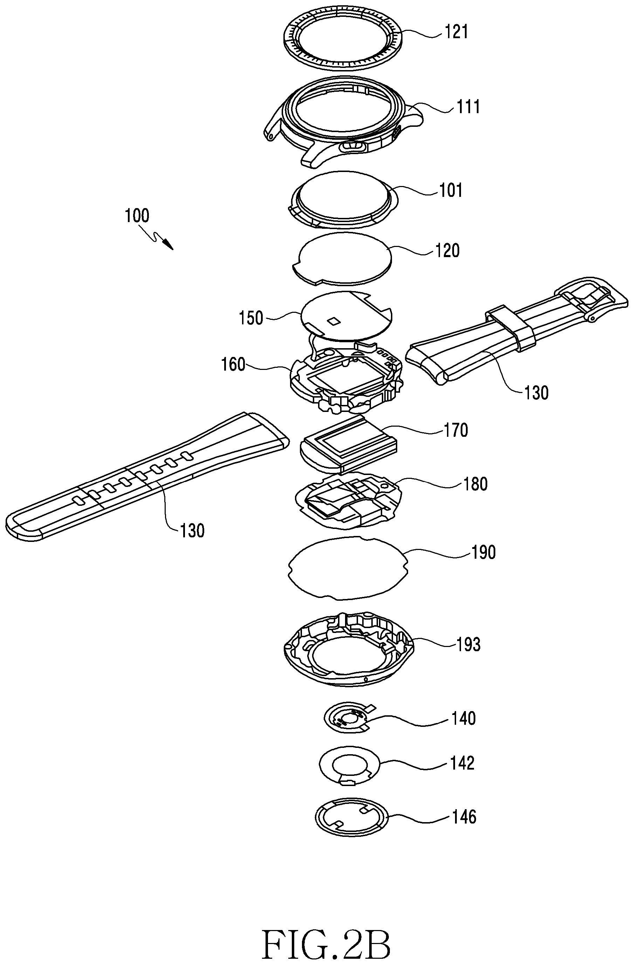

According to an embodiment, the housing 110 may include a first surface facing a first direction, a second surface facing a second direction opposite to the first direction, and a lateral surface configured to surround at least a portion of a space between the first surface and the second surface. According to an embodiment, two or more of the first surface, the second surface, and the lateral surface of the housing 110 may be integrally formed. According to an embodiment, the housing 110 may be configured by various combinations. For example, the housing 110 may be configured by a combination of a lateral bezel structure 111 and a rear plate 193 in FIG. 2B. According to an embodiment, the first surface of the housing 110 may mean a surface including at least one of a first electrode 201 and a second electrode 202, and the second surface and/or the lateral surface of the housing 110 may mean a surface including at least one of a third electrode 203 and a fourth electrode 204.

According to an embodiment, the electrode 201, 202, 203, or 204 may be exposed to the outside through at least a portion of the housing 110. For example, at least one of the first electrode 201 and the second electrode 202 may be exposed to the outside through at least a portion of the first surface of the housing. Furthermore, at least one of the third electrode 203 and the fourth electrode 204 may be exposed to the outside through at least a portion of the second surface and/or the lateral surface of the housing. The electrode 201, 202, 203, or 204 may be formed of a conductive material through which a current may flow. The shape and size of the electrode may vary. In addition, at least one electrode included in the wearable device 100 may exist on the first surface and the second surface of the wearable device 100, or on a portion of the housing 110 rather than the first surface and the second surface.

According to an embodiment, the display 120 may visually provide biometric data of a user to the user of the wearable device 100. According to an embodiment, the display 120 may convert an output screen through an input of the user with respect to a portion (e.g., a bezel) of the housing 110. For example, the display 120 may convert a watch screen into a biometric data screen (e.g., a body component and a heart rate) in response to a user input.

According to an embodiment, the strap 130 may be connected to at least a portion of the housing 110 and detachably couple the wearable device 100 to a portion (e.g., a wrist, an ankle, and the like) of the user body. According to an embodiment, the user of the wearable device 100 may adjust the strap 130 to increase a degree of adhesion.

FIG. 2B is a perspective view illustrating a wearable device according to an embodiment.

Referring to FIG. 2B, the electronic device 100 may include a lateral bezel structure 111, a wheel key 121, a front plate 101, a display 120, an antenna 150, a support member 160(e.g., a bracket), a battery 170, a printed circuit board 180, a sealing member 190, a rear plate 193, a biometric sensor 140, a wireless charging coil 142, an electrode 146, and a strap 130. The support member 160 may be disposed in the electronic device 100 to be connected to the lateral bezel structure 111 or integrally formed with the lateral bezel structure 111. The support member 160 may be formed of, for example, a metal material and/or a non-metal (e.g., polymer) material. The support member 160 may have the display 120 coupled to one surface thereof and the printed circuit board 180 coupled to the other surface thereof. A processor, a memory, and/or an interface may be mounted to the printed circuit board 180. The processor may include, for example, one or more of a central processing unit, an application processor, a graphic processing unit (GPU), an application processor, a sensor processor, or a communication processor.

The memory may include, for example, a volatile memory or a non-volatile memory. The interface may include, for example, a high-definition multimedia interface (HDMI), a universal serial bus (USB) interface, an SD card interface, and/or an audio interface. The interface, for example, may electrically or physically connect the electronic device 100 to an external electronic device, and may include a USB connector, an SD card/MMC connector, or an audio connector.

The battery 170 is a device for supplying power to at least one component of the electronic device 100, and may include, for example, a non-rechargeable primary battery, or a rechargeable secondary battery, or a fuel cell. At least a part of the battery 170 may be disposed on the substantially same plane as the printed circuit board 180. The battery 170 may be disposed and integrally formed in the electronic device 100 or may be disposed to be attachable to/detachable from the electronic device 100.

The antenna 150 may be disposed between the display 120 and the support member 160. The antenna 150 may include, for example, a near field communication (NFC) antenna, a wireless charging antenna, and/or a magnetic secure transmission (MST) antenna. The antenna 150, for example, may perform a near field communication with an external electronic device, wirelessly transmit and receive power required for charging, or transmit a magnetism-based signal including a near field communication signal or payment data. In another embodiment, an antenna structure may be formed of a part or a combination of the lateral bezel structure 111 and/or the support member 160.

The sealing member 190 may be disposed between the lateral bezel structure 111 and the rear plate 193. The sealing member 190 may be configured to block moisture and foreign substances from being introduced from the outside to a space surrounded by the lateral bezel structure 111 and the rear plate 193.

The biometric sensor 140 and the wireless charging coil 142 may be located between the rear plate 193 and the battery 146. In another embodiment, the biometric sensor 140 and the wireless charging coil 142 may also be located between the sealing member 190 and the electrode 146.

The electrode 146 may be exposed to the outside through the first surface of the housing 110. According to an embodiment, the electrode 146 may include two or more electrodes electrically separated from each other.

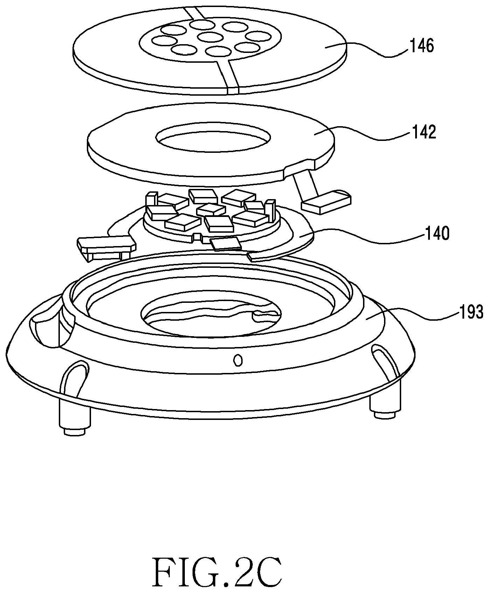

FIG. 2C is an exploded view illustrating a lower surface of a wearable device according to an embodiment.

Referring to FIG. 2C, a rear plate 193, an electrode 146, a biometric sensor 140, and a wireless charging coil 142 may be located on a lower surface of an electronic device (e.g., the electronic device 100 in FIG. 2B).

According to an embodiment, the electrode 146 may be electrically connected to the biometric sensor 140 included in the electronic device 100 and used for acquiring body information or health information. For example, the electrode 146 may be electrically connected to a bioelectric impedance analysis (BIA) sensor included in the electronic device 100 and used for measuring a body fat ratio. Furthermore, the electrode may be electrically connected to an electrocardiogram (ECG) sensor included in the electronic device and used for measuring electrocardiogram. The sensor connectible to the electrode 146 is merely exemplary and is not limited thereto.

The structure of the above-described wearable device 100 is exemplary and the wearable device 100 may be implemented differently from FIG. 2A, FIG. 2B, and FIG. 2C. The wearable device 100 may have various shapes/structures suitable for performing the method for measuring biometric data disclosed herein.

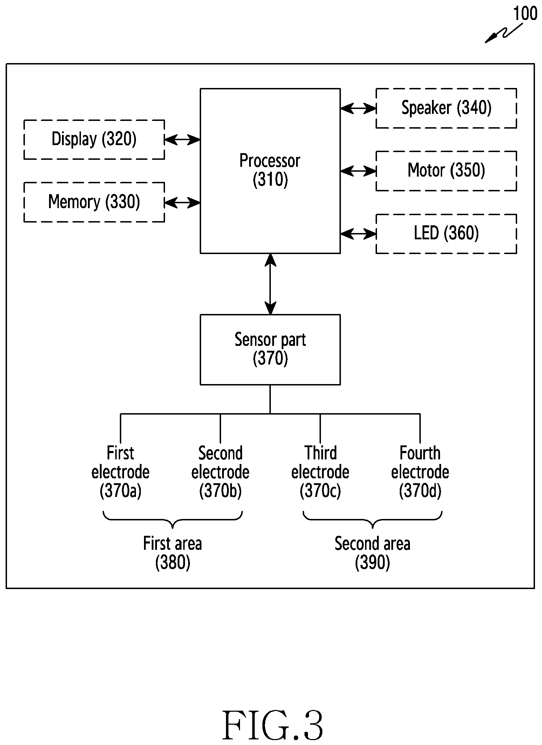

FIG. 3 is a block diagram illustrating a wearable device according to an embodiment.

Referring to FIG. 3, the wearable device 100 may include a processor 310, a display 320, a memory 330, a speaker 340, a motor 350, an LED 360, and a sensor part 370. In various embodiments, the wearable device 100 may additionally include components other than the component described in FIG. 3 or omit at least one of the components described in FIG. 3.

According to an embodiment, the processor 310 may acquire biometric data (e.g., impedance phase information) of a user through a data value (e.g., a body component) acquired from the sensor part 370. According to an embodiment, the display 310 may provide the acquired biometric data to the user through the display 320. According to an embodiment, in case that the acquired biometric data deviates from a predetermined range, the processor 310 may provide a guide for inducing remeasurement to the user through the display 320.

According to an embodiment, the memory 330 may store various data used by at least one component (e.g., a processor) of the electronic device 100. For example, the memory 330 may store user biometric data acquired by the sensor part 370.

According to an embodiment, the speaker 340 may output audio data stored in the memory 330, based on the user biometric data acquired by the sensor part 370. For example, in case that the acquired biometric data deviates from a predetermined range, the processor 310 may output a voice guide or sound for providing a notification related to remeasurement to the user through the speaker 340.

According to an embodiment, the motor 350 may convert an electrical signal into a mechanical stimulation (e.g., vibration or movement) that a user may perceive. For example, in case that the user's wearing is detected, but both hands of a user are into contact, or it is determined that the electrode has an error, the processor 310 may output vibration through the motor 350.

According to an embodiment, the LED 360 may emit light having a type that a user may perceive, based on an electrical signal received from the biometric sensor. For example, in case that the user's wearing is detected, but both hands of the user are in contact, or it is determined that the electrode has an error, the processor 310 may provide a notification to a user through flashing of the LED.

According to an embodiment, the sensor part 370 may detect a state of a user and transmit a signal corresponding to the detected state to the processor 310. According to an embodiment, the sensor part 370 may include a biometric sensor (e.g., the biometric sensor 140 in FIG. 2B). According to an embodiment, the biometric sensor may include at least one of a bioelectrical impedance analysis (BIA) sensor, a heart ratio monitor (HRM) sensor, an electrocardiogram (ECG) sensor, and a saturation of percutaneous oxygen (Sp02) sensor.

According to an embodiment, the sensor part 370 may include multiple electrodes. According to an embodiment, the multiple electrodes may include a first electrode, a second electrode, a third electrode, and a fourth electrode. According to an embodiment, the first electrode and the second electrode may come into contact with a first area 380 of a user body. According to an embodiment, the third electrode and the fourth electrode may come into contact with a second area 390 of a user body, which does not overlap the first area 380.

The sensor part 370 disclosed herein may be referred to as simply a (at least one) sensor, a sensor circuit, a sensor module, or the like.

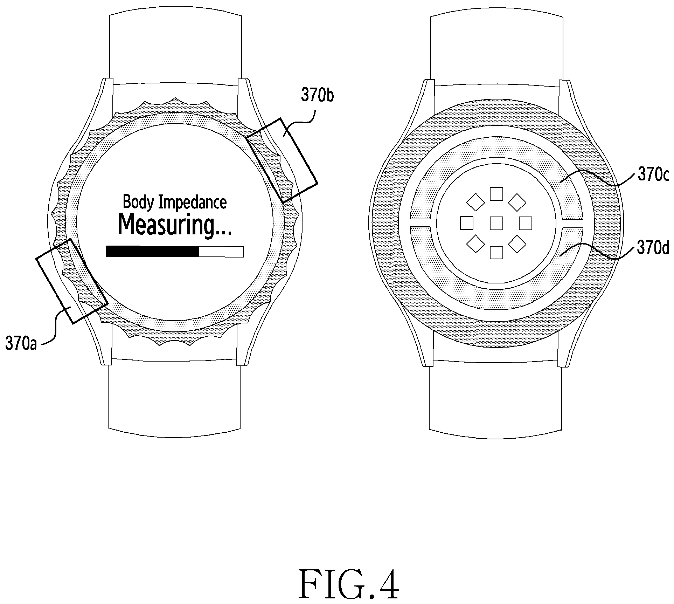

FIG. 4 is a view illustrating a location of an electrode in a wearable device according to an embodiment.

According to an embodiment, the BIA sensor (e.g., the biometric sensor 140 in FIG. 2B or the sensor part 370 in FIG. 3) may include multiple electrodes. According to an embodiment, a first electrode 370a and a second electrode 370b of the electrodes of the BIA sensor may be arranged on an upper surface portion or lateral surface portion of the wearable device 100. According to an embodiment, a third electrode 370c and a fourth electrode 370d may be arranged on a rear surface portion of the wearable device not to overlap the first electrode 370a and the second electrode 370b. According to an embodiment, the first electrode 370a, the second electrode 370b, the third electrode 370c, and the fourth electrode 370d may be exposed to the outside of the housing while being spaced apart from each other.

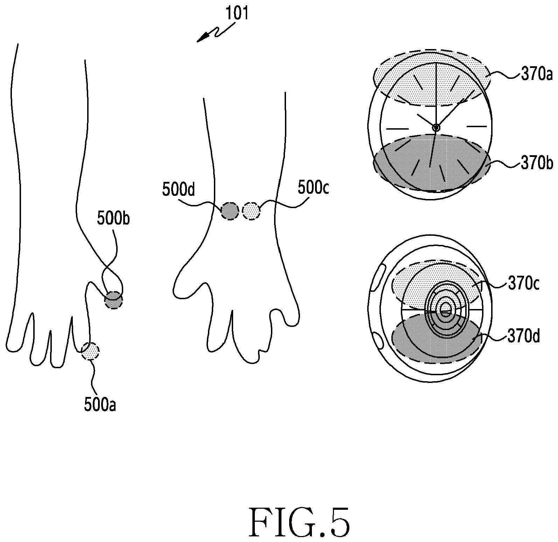

FIG. 5 is a view illustrating locations of an electrode and a user body coming into contact with the electrode in a wearable device according to an embodiment.

According to an embodiment, the first electrode 370a of the biometric sensor may come into contact with a portion 500a of a right hand (or a left hand) of the user 101, the second electrode 370b may come into contact with a portion 500b of the right hand of the user 101 not to overlap the portion 500a of the right hand (or the left hand) of the user.

According to an embodiment, the third electrode 370c of the biometric sensor may come into contact with a portion 500c of the left hand (or the right hand) of the user and the fourth electrode 370d may come into contact with a portion 500d of the left hand (or the right hand).

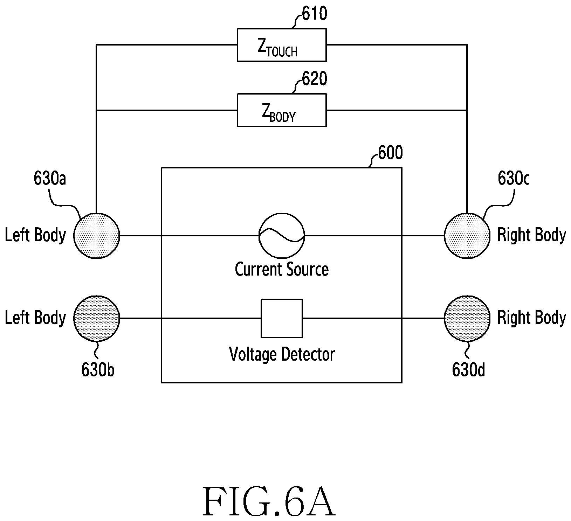

FIG. 6A is a view illustrating a human body closed loop formed in case that a user body comes into contact with an electrode connected to a biometric sensor in a wearable device according to an embodiment.

Referring to FIG. 6A, a first electrode 630a (e.g., the first electrode 370a in FIG. 3) of the biometric sensor 600 (e.g., the biometric sensor 140 in FIG. 2B) according to an embodiment may be connected to an alternating current source. According to an embodiment, a third electrode 630c (e.g., the second electrode 370b in FIG. 3) of the biometric sensor 600 may be connected to an alternating current source. According to an embodiment, in case that a portion of the user body comes into contact with the first electrode 630a and the third electrode 630c, a micro current may flow to the human body. According to an embodiment, in case that the micro current flows to the human body, a current loop 620 may be formed between the contacted portions. According to an embodiment, in case that body portions respectively in contact with the first electrode 630a and the third electrode come into contact with each other, an unexpected human body closed loop 610 may be formed. For example, in case that the right hand and the left hand of a user come into contact with each other, a different human body closed loop 610 may be formed so that data that may not be generated from the human body in a correct posture may be measured.

According to an embodiment, a second electrode 630b (e.g., the second electrode 370b in FIG. 3) of the biometric sensor 600 may be connected to a voltage detector. According to an embodiment, a fourth electrode 630d (e.g., the fourth electrode 370d in FIG. 3) of the biometric sensor 600 may be connected to a voltage detector. The voltage detector connected to the second electrode 630b and the fourth electrode 630d may measure a voltage between both electrodes.

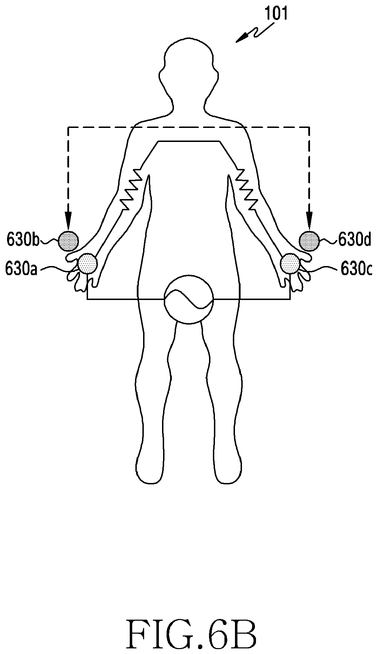

FIG. 6B is a view illustrating, on a human body, a human body closed loop formed in case that a user body comes into contact with an electrode connected to a biometric sensor in a wearable device according to an embodiment.

According to an embodiment the first electrode 630a and the third electrode 630c may be electrodes for supplying a current. According to an embodiment the second electrode 630b and the fourth electrode 630d may be electrodes for measuring a voltage. According to an embodiment, in case that a user body comes into contact with the first electrode and the third electrode, a human body closed loop may be formed. According to an embodiment, in case that the human body closed loop is formed, a value corresponding to a sum of impedances of the left arm and the right arm may be measured.

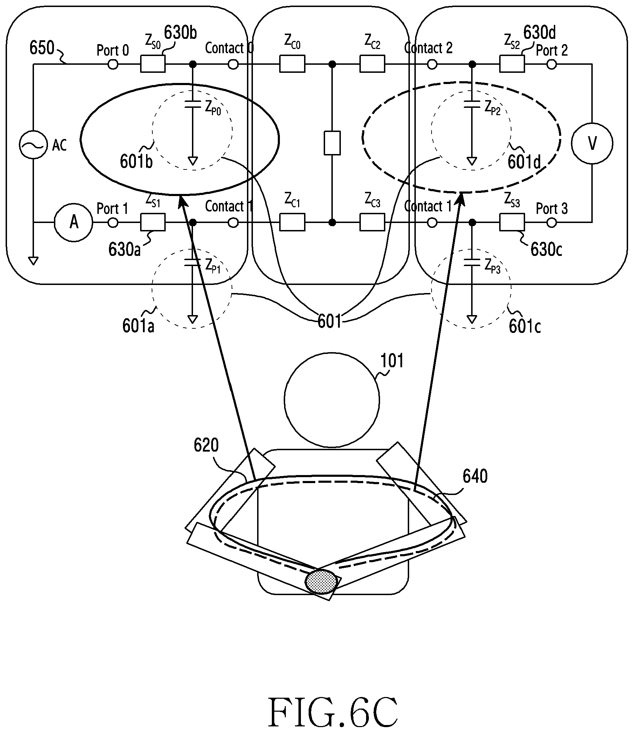

FIG. 6C is a view illustrating acquisition of biometric data considering of a parasitic component existing in a biometric contact circuit in a wearable device according to an embodiment.

Referring to FIG. 6C, the processor 310 according to an embodiment may measure a parasitic impedance value 601 through the biometric circuit 650. The parasitic impedance value 601 may be produced with respect to four electrodes (e.g., the first electrode 630a, the second electrode 630b, the third electrode 630c, and the fourth electrode 630d) included in the biometric circuit 650. For example, a first parasitic impedance value 601a, a second parasitic impedance value 601b, a third parasitic impedance value 601c, and a fourth parasitic impedance value 601d may be produced with respect to the first electrode 630a, the second electrode 630b, the third electrode 630c, and the fourth electrode 630d, respectively.

According to an embodiment, the parasitic impedance value 601 may mean a value derived from a parasitic component inside the wearable device. For example, the parasitic component may include at least one of unintentionally produced resistance, capacitance, inductance, and/or a component that interfere with an electrical signal in the biometric circuit 650.

According to an embodiment, the processor 310 may store the parasitic impedance value 601 measured through the biometric circuit 650 in the memory. According to an embodiment, the processor 310 may detect, through the biometric circuit 650, that a first portion of the human body of the user 101 comes into contact with the first electrode 630a and the second electrode 630b and a second portion of the human body of the user 101 comes into contact with the third electrode 630c and the fourth electrode 630d.

According to an embodiment, the processor 310 may apply a current to the human body through the second electrode 630b and the third electrode 630c via the biometric circuit 650. According to an embodiment, in case that a portion of the user (101) body comes into contact with the first electrode 630a and the third electrode 630c, a micro current may flow to the human body. According to an embodiment, in case that the micro current flows to the human body, a current loop 620 may be formed between the contacted portions. According to an embodiment, the current may flow through the current loop 620.

According to an embodiment, the processor 310 may measure a voltage across both ends of the first electrode 630a and the fourth electrode 630d through the biometric circuit 650 in response to the application of the current. According to an embodiment, a voltage loop 640 in which voltage is measured may be formed in the human body.

According to an embodiment, the processor 310 may acquire impedance phase information of the human body based on the applied current, the measured voltage, and the parasitic impedance value 601.

According to an embodiment, in case that the acquired impedance information deviates from a predetermined range, the processor 310 may be configured to provide a notification related to the biometric data measurement.

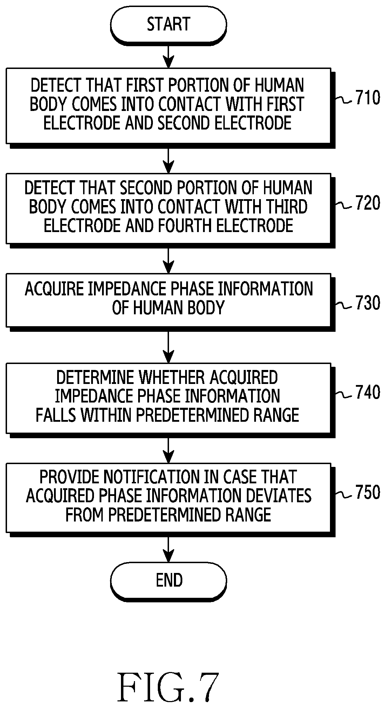

FIG. 7 is a flowchart illustrating a content for providing a notification related to biometric data measurement to a user according to acquired biometric data in a wearable device according to an embodiment.

According to an embodiment, in operation 710, the wearable device (e.g., the wearable device 100 in FIG. 1) may detect, through a biometric sensor, that a first portion of a human body comes into contact with a first electrode and a second electrode.

According to an embodiment, in operation 720, the wearable device 100 may detect, through the biometric sensor, that a second portion of the human body comes into contact with a third electrode and a fourth electrode.

According to an embodiment, in case of detecting a measurement mode, the wearable device 100 may detect that the first portion of the human body comes into contact with the first electrode and the second electrode and that the second portion of the human body comes into contact with the third electrode and the fourth electrode so as to measure biometric information. According to an embodiment, in case of detecting that the first portion of the human body comes into contact with the first electrode and the second electrode and that the second portion of the human body comes into contact with the third electrode and the fourth electrode, the wearable device 100 may automatically measure biometric information. According to an embodiment, the biometric information may include impedance phase information and/or impedance size information.

According to an embodiment, in operation 730, the wearable device 100 may acquire impedance phase information of the human body by using Equation 1, Equation 2, and Equation 3.

【Equation 1】

$$Z = R + jX$$

In Equation 1, Z indicates a bio-impedance value. R indicates a resistance component and X indicates a reactance component. The impedance phase information is acquired by using the reactance component.

【Equation 2】

$$V = IR$$

In Equation 2, V indicates a voltage value measured by a voltage detector. I indicates a current value, which flows through a human body from an alternating current source. R indicates a resistance component. The resistance component is acquired by using the voltage value and the current value.

【Equation 3】

$$phase\ angle = \arctan(\frac{XL-XC}{R})$$

In Equation 3, a phase angle may mean a phase angle difference between a voltage and a current. $X_L$ indicates an inductive reactance, and Xc means a capacitive reactance. The phase angle is acquired by using information of the inductive reactance and/or the capacitive reactance. For example, a value of the capacitive reactance may be acquired by using a value of a voltage at an electrode and a value of a reference capacitance inside a circuit. As shown in the above-described embodiment, the phase angle may be acquired by using information of the inductive reactance.

According to an embodiment, in operation 740, the wearable device 100 may determine whether the acquired impedance phase information acquired using Equation 2 and Equation 3 through the processor falls within a predetermined range.

According to an embodiment, in case that the acquired impedance phase information falls within a predetermined range, the wearable device 100 may provide a UI indicating that measurement is normally performed to a user through a display.

According to an embodiment, in operation 750, in case that acquired phase information deviates a predetermined range, the wearable device 100 may provide a notification to a user. According to another embodiment, in case that acquired impedance size information deviates a predetermined range, the wearable device 100 may provide a notification to a user.

According to an embodiment, in case that the acquired phase information falls within a first range, the wearable device 100 may provide a notification with respect to a measurement error. For example, the measurement error may include at least one of a case in which user's information is insufficiently input, a case in which a user's wrist is not into contact with an electrode, a case in which a user's finger is not in contact with an electrode, a case in which a user's wrist is dry enough to prevent a current from flowing, a case in which a user's finger is dry enough to prevent a current from flowing, a case in which a user's both hands are in contact, and a case in which a user's posture is not stable.

According to an embodiment, in case that the acquired phase information falls within a second range, the wearable device 100 may provide a notification with respect to an electrode error. According to an embodiment, the electrode error may include a case in which an electrode is short-circuiting or grounded.

According to an embodiment, the notification provided to the user may include at least one of a UI through a display, a voice guide or sound through a speaker, vibration through a motor, and flashing of an LED. For example, in case that the user's information is insufficiently input, a UI for guiding to input user's information may be provided together with vibration.

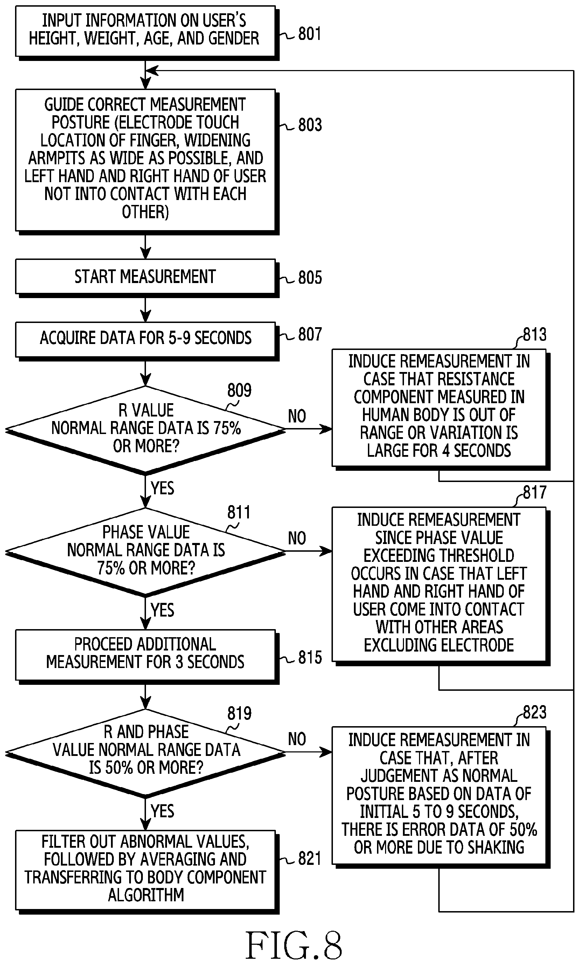

FIG. 8 is a flowchart for selecting a control method of a processor according to whether acquired biometric data is within a predetermined range in a wearable device according to an embodiment. In relation to a description of FIG. 8, a content corresponding to, identical to, or similar to the aforementioned description may be omitted.

Referring to FIG. 8, in operation 801, a user (e.g., the user 101 in FIG. 1) may input information such as user's height, weight, age, and gender to a wearable device (e.g., the electronic device 100 in FIG. 1). According to an embodiment, the user's information may be stored in a memory (e.g., the memory 330 in FIG. 3).

According to an embodiment, in operation 803, the wearable device 100 may provide a guide for a correct measurement posture to the user 101. For example, the wearable device may inform the user of a location in which a finger touches an electrode and guide to take a posture in which the armpits are widen as wide as possible and the left and right hand of a user do not touch with each other.

According to an embodiment, in operation 805, the wearable device 100 may start bio-impedance measurement of the user 101. According to an embodiment, in case of detecting a measurement mode, the wearable device 100 may detect whether a human body of the user 101 comes into contact with an electrode and start measurement. According to an embodiment, in case of detecting whether a human body of the user 101 comes into contact with an electrode, the wearable device 100 may automatically start measurement.

According to an embodiment, in operation 807, the wearable device 100 may acquire biometric data during a first time through a sensor part (e.g., the sensor part 370 in FIG. 3). For example, the wearable device 100 may acquire biometric data for 5 to 9 seconds. According to an embodiment, the biometric data may include a heart rate, blood oxygen saturation (Sp02), electrocardiogram, and bio-impedance information.

According to an embodiment, in operation 809, the wearable device 100 may determine whether a resistance value of the bio-impedance of the user 101 falls within a predetermined range when determined based on first accuracy. For example, it may be determined whether the user's body resistance falls within a predetermined range by counting frequencies at which the human body resistance measured during a first time falls within a certain range (e.g., +/- 50$\Omega$) with reference to 700 $\Omega$.

According to an embodiment, it may be determined that the resistance value of the bio-impedance falls within a predetermined range when determined based on the first accuracy. For example, it may be determined that the user's body resistance falls within a predetermined range in case that the frequency at which the user's body resistance measured during the first time is within a certain range (e.g., +/- 50$\Omega$) with reference to 700 $\Omega$ is 75% or more of the total samples. Accordingly, in operation 811, the wearable device may determine whether phase information of the bio-impedance falls within a predetermined range based on the first accuracy.

According to an embodiment, it may be determined that the resistance value of the bio-impedance deviates from a predetermined range when determined based on the first accuracy. For example, in case that a resistance variation measured during a first time is larger than or equal to a designated reference, it may be determined to deviate from a predetermined range. Accordingly, in operation 813, the wearable device may induce remeasurement of the user through a guide. For example, the guide for remeasurement may include at least one of a UI through a display, a voice guide or sound through a speaker, vibration through a motor, and flashing of an LED.

According to an embodiment, in operation 811, the wearable device may determine that the resistance value of the bio-impedance falls within a predetermined range when determined based on the first accuracy. Accordingly, in operation 815, the wearable device may additionally proceed bio-impedance measurement during a second time. For example, the wearable device may additionally proceed bio-impedance measurement for 3 seconds.

According to an embodiment, the wearable device may determine that the resistance value of the bio-impedance deviates from a predetermined range when determined based on the first accuracy. Accordingly, in operation 817, the wearable device may induce remeasurement of the user through a guide.

According to an embodiment, in operation 819, the wearable device may determine whether phase information and a resistance value of the bio-impedance fall within a predetermined range when determined based on the second accuracy.

According to an embodiment, it may be determined that the phase information and resistance value of the bio-impedance falls within a predetermined range when determined based on the second accuracy. For example, it may be determined that the user's body resistance falls within a predetermined range in case that the frequency at which the user's body resistance measured during the second time is within a certain range (e.g., +/- 50Ω) with reference to 700 Ω is 50% or more of the total samples. Accordingly, in operation 821, the wearable device may filter out values deviating from a predetermined range and calculate an average value from values within a predetermined range to be substituted into a body component algorithm.

According to an embodiment, the wearable device may determine that the resistance value and phase information of the bio-impedance deviate from a predetermined range when determined based on the second accuracy. Accordingly, in operation 823, the wearable device may induce remeasurement of the user.

According to an embodiment, the first accuracy and/or the second accuracy may be determined based on the biometric information acquired from the user. For example, the biometric information may include at least one of a magnitude of the user's bio-impedance, a resistance value of the user's bio-impedance, and a reactance value of the user's bio-impedance.

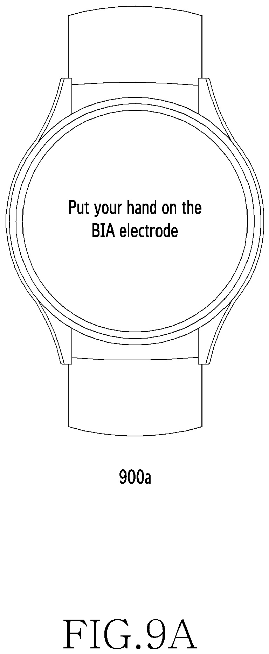

FIG. 9A to FIG. 9E are views illustrating UIs displayed on a display in a wearable device according to various embodiments. In relation to a description of FIG. 9A to FIG. 9E, a content corresponding to, identical to, or similar to the aforementioned description may be omitted.

Referring to FIG. 9A, the wearable device 900a (e.g., the wearable device 100 in FIG. 1) according to an embodiment may stop measuring a bio-impedance in case that a user (e.g., the user 101 in FIG. 1) does not bring a human body in contact with an electrode at a correct position. According to an embodiment, in case of determining that a user does not bring a human body in contact with an electrode at a correct position, the wearable device 900a may output a guide message to the user through a display. For example, the wearable device 900a may output a guide message (e.g., "Put your hand on the BIA electrode") for guiding the user to bring a finger in contact with a correct location.

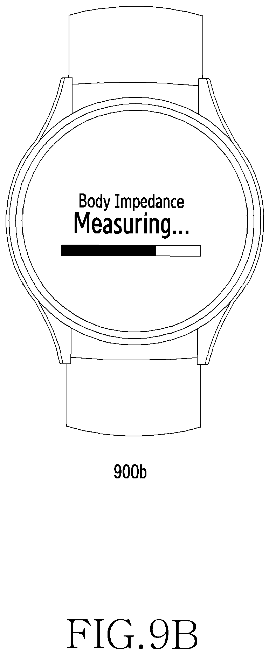

Referring to FIG. 9B, in case of determining that the bio-impedance of the user is normally measured, the wearable device 900b according to an embodiment may continuously proceed the bio-impedance measurement. According to an embodiment, in case of determining that the bio-impedance of the user is normally measured, the wearable device 900b may output a guide message to the user through a display. For example, the wearable device 900b may output a guide message (e.g., "Body impedance measuring...") to inform that the bio-impedance of the user is normally measured.

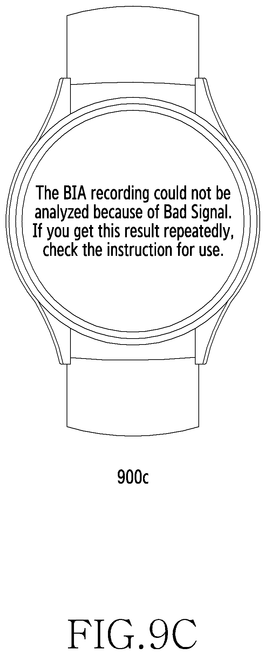

Referring to FIG. 9C, in case of determining that the bio-impedance of the user is not normally measured, the wearable device 900c according to an embodiment may stop the bio-impedance measurement. For example, the case of determining that the bio-impedance of the user is not normally measured may include a case of not receiving a measured signal or a case that a measured signal is received but deviate from a normal range. In another embodiment, the case of determining that the bio-impedance of the user is not normally measured may include at least one of a case that the user's hand is not in contact with the electrode, a case that only one hand is in contact with the electrode, a case that the user's hands are in contact with each other, a case that the user's finger or wrist is dry enough to make it difficult to measure normal biometric information, and a case that biometric information may not be measured due to the user's movement.

According to an embodiment, in case of determining that an electrode has an error, the wearable device 900c may output a guide message to the user through a display. For example, the wearable device 900c may output a guide message (e.g., "There is an error and proper function is not possible") indicating that the electrode has an error.

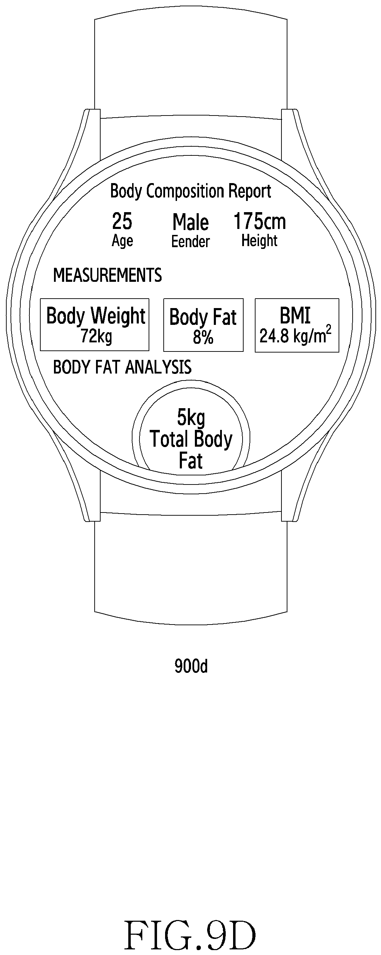

Referring to FIG. 9D, in case that the bio-impedance has been normally measured, the wearable device 900d according to an embodiment may provide a content related to a biometric analysis result to a user through a display. For example, the biometric analysis result may include information related to at least one of the user's weight, muscle mass, body fat mass, body fat percentage, and BMI. In another embodiment, the wearable device 900d may provide information indicating whether the biometric analysis result of the user falls within a reference range through a display.

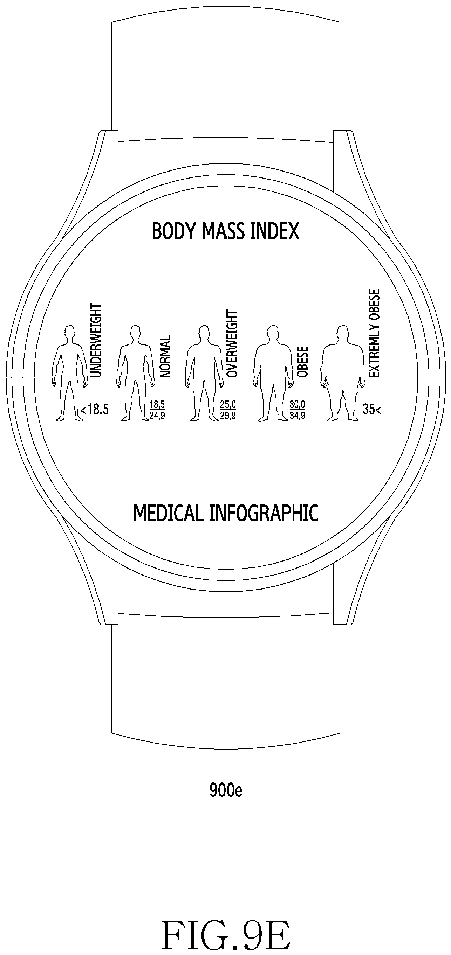

Referring to FIG. 9E, in case that the bio-impedance has been normally measured, the wearable device 900e according to an embodiment may provide a content related to a biometric analysis result in a graphic form to a user through a display. For example, the wearable device 900e may provide information indicating whether the user has a standard weight in a graphic form, based on information on at least one of the user's weight, muscle mass, body fat mass, body fat percentage, and BMI.

According to an embodiment, the wearable device 900 may output notification vibration to induce the user to remeasure through a haptic motor (not shown). For example, the wearable device 900 may provide, to the user, a guide message through a display and/or notification vibration through a haptic motor so as to cause the user to take a correct posture.

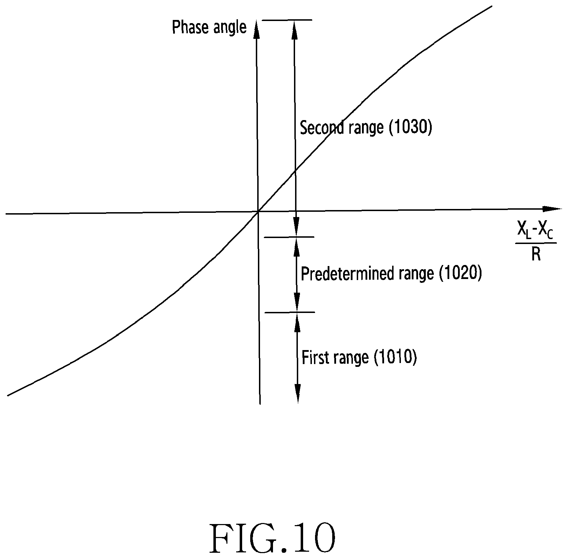

FIG. 10 illustrates a graph indicating a method for determining whether acquired biometric data is within a predetermined range in a wearable device according to an embodiment. In relation to a description of FIG. 10, a content corresponding to, identical to, or similar to the aforementioned description may be omitted.

Referring to FIG. 10, the wearable device (e.g., the electronic device 100 in FIG. 1) may detect that impedance phase information of a user falls in a first range 1010, a predetermined range 1020, and a second range 1030. According to an embodiment, in case that phase information falls within the first range 1010, a processor (e.g., 310 in FIG. 3) of the wearable device may control to provide a guide for remeasurement to a user through at least one of a display, a motor, a speaker, and an LED.

According to an embodiment, in case that phase information falls within the predetermined range 1020, the processor (310 in FIG. 3) of the wearable device may control a biometric sensor to continuously measure bio-impedance of a user.

According to an embodiment, in case that phase information falls within the second range 1030, the processor (e.g., 310 in FIG. 3) of the wearable device may control to provide a notification indicating that an electrode has an error to a user through at least one of a display, a motor, a speaker, and an LED.

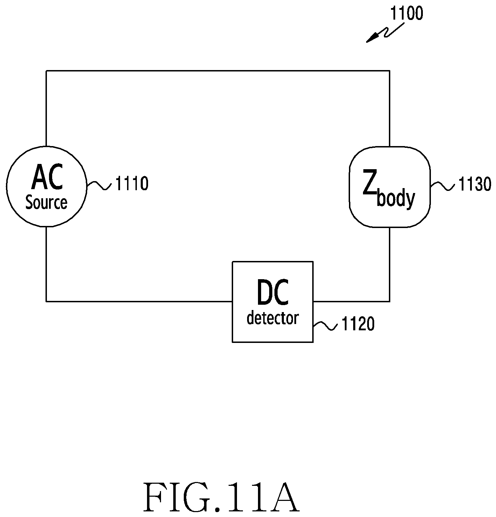

FIG. 11A is a view illustrating a human body closed loop formed in case that an acquired phase information falls within a predetermined range in a wearable device according to an embodiment.

According to an embodiment, a bio-impedance value may be derived by information on a current flowing to the human body through an alternating current source 1110 and information on a voltage measured by a voltage detector 1120. According to an embodiment, the phase information may be derived from a value including reactance other than a resistance value in the bio-impedance. According to an embodiment, in case that an alternating current flows through an alternating current source 1110, a human body closed loop 1100 through both arms of a user may be formed. According to an embodiment, in case that a normal human body closed loop is formed, a normal bio-impedance 1130 may be measured. For example, in case that a normal human body closed loop is formed, a phase angle may be within a range of -3° to -14°.

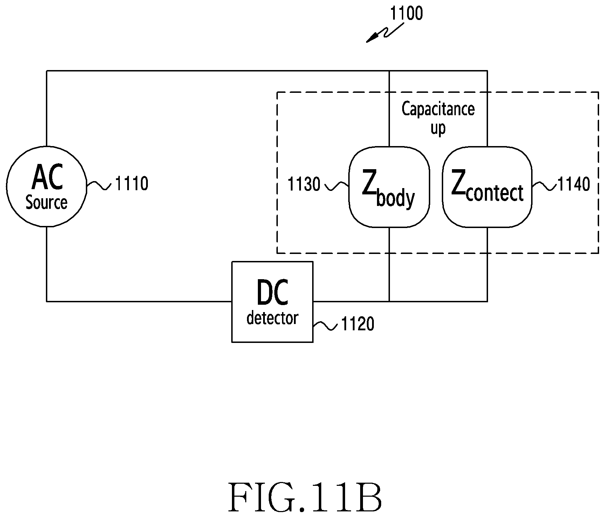

FIG. 11B is a view illustrating a human body closed loop formed in case that both hands of a user are in contact in a wearable device according to an embodiment.

According to an embodiment, in case that a closed loop caused by contact of both hands of a user is formed in addition to the normal human body closed loop, a bio-impedance 1140 caused by the contact of both hands other than the normal human body closed loop 1130 may be measured. For example, in case of measuring a bio-impedance in a state of contacting both hands of a user, a phase angle may be within a range below -14°.

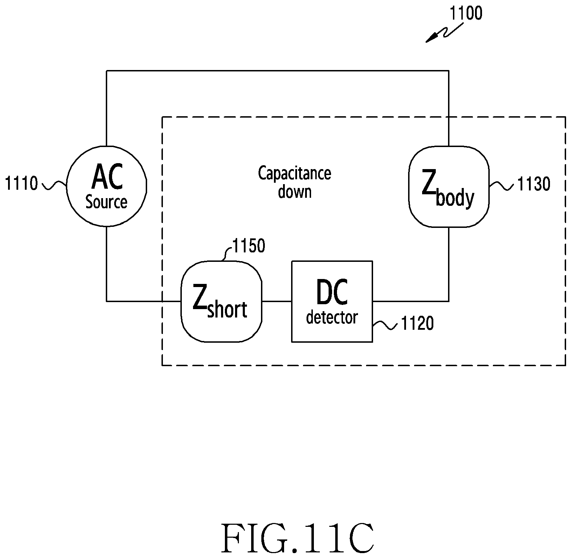

FIG. 11C is a view illustrating a human body closed loop formed in case that an error occurs in an electrode in a wearable device according to an embodiment.

According to an embodiment, in case that an electrode has an error, a bio-impedance 1150 caused by the error of the electrode other than the normal human body closed loop 1130 may be measured. For example, a phase angle acquired in case that the electrode is short-circuited or grounded may be a range above -3°.

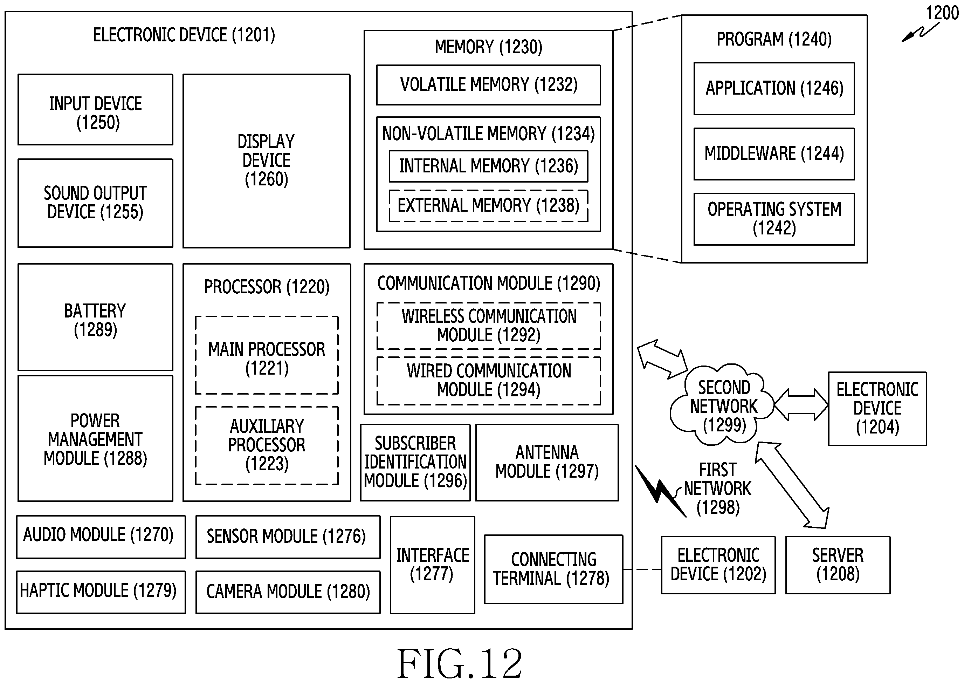

Fig. 12 is a block diagram illustrating an electronic device 1201 in a network environment 1200 according to various embodiments. Referring to Fig. 12, the electronic device 1201 in the network environment 1200 may communicate with an electronic device 1202 via a first network 1298 (e.g., a short-range wireless communication network), or at least one of an electronic device 1204 or a server 1208 via a second network 1299 (e.g., a long-range wireless communication network). According to an embodiment, the electronic device 1201 may communicate with the electronic device 1204 via the server 1208. According to an embodiment, the electronic device 1201 may include a processor 1220, memory 1230, an input module 1250, a sound output module 1255, a display module 1260, an audio module 1270, a sensor module 1276, an interface 1277, a connecting terminal 1278, a haptic module 1279, a camera module 1280, a power management module 1288, a battery 1289, a communication module 1290, a subscriber identification module(SIM) 1296, or an antenna module 1297. In some embodiments, at least one of the components (e.g., the connecting terminal 1278) may be omitted from the electronic device 1201, or one or more other components may be added in the electronic device 1201. In some embodiments, some of the components (e.g., the sensor module 1276, the camera module 1280, or the antenna module 1297) may be implemented as a single component (e.g., the display module 1260).

The processor 1220 may execute, for example, software (e.g., a program 1240) to control at least one other component (e.g., a hardware or software component) of the electronic device 1201 coupled with the processor 1220, and may perform various data processing or computation. According to one embodiment, as at least part of the data processing or computation, the processor 1220 may store a command or data received from another component (e.g., the sensor module 1276 or the communication module 1290) in volatile memory 1232, process the command or the data stored in the volatile memory 1232, and store resulting data in non-volatile memory 1234. According to an embodiment, the processor 1220 may include a main processor 1221 (e.g., a central processing unit (CPU) or an application processor (AP)), or an auxiliary processor 1223 (e.g., a graphics processing unit (GPU), a neural processing unit (NPU), an image signal processor (ISP), a sensor hub processor, or a communication processor (CP)) that is operable independently from, or in conjunction with, the main processor 1221. For example, when the electronic device 1201 includes the main processor 1221 and the auxiliary processor 1223, the auxiliary processor 1223 may be adapted to consume less power than the main processor 1221, or to be specific to a specified function. The auxiliary processor 1223 may be implemented as separate from, or as part of the main processor 1221.

The auxiliary processor 1223 may control at least some of functions or states related to at least one component (e.g., the display module 1260, the sensor module 1276, or the communication module 1290) among the components of the electronic device 1201, instead of the main processor 1221 while the main processor 1221 is in an inactive (e.g., sleep) state, or together with the main processor 1221 while the main processor 1221 is in an active state (e.g., executing an application). According to an embodiment, the auxiliary processor 1223 (e.g., an image signal processor or a communication processor) may be implemented as part of another component (e.g., the camera module 1280 or the communication module 1290) functionally related to the auxiliary processor 1223. According to an embodiment, the auxiliary processor 1223 (e.g., the neural processing unit) may include a hardware structure specified for artificial intelligence model processing. An artificial intelligence model may be generated by machine learning. Such learning may be performed, e.g., by the electronic device 1201 where the artificial intelligence is performed or via a separate server (e.g., the server 1208). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 1230 may store various data used by at least one component (e.g., the processor 1220 or the sensor module 1276) of the electronic device 1201. The various data may include, for example, software (e.g., the program 1240) and input data or output data for a command related thererto. The memory 1230 may include the volatile memory 1232 or the non-volatile memory 1234.

The program 1240 may be stored in the memory 1230 as software, and may include, for example, an operating system (OS) 1242, middleware 1244, or an application 1246.

The input module 1250 may receive a command or data to be used by another component (e.g., the processor 1220) of the electronic device 1201, from the outside (e.g., a user) of the electronic device 1201. The input module 1250 may include, for example, a microphone, a mouse, a keyboard, a key (e.g., a button), or a digital pen (e.g., a stylus pen).

The sound output module 1255 may output sound signals to the outside of the electronic device 1201. The sound output module 1255 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 1260 may visually provide information to the outside (e.g., a user) of the electronic device 1201. The display module 1260 may include, for example, a display, a hologram device, or a projector and control circuitry to control a corresponding one of the display, hologram device, and projector. According to an embodiment, the display module 1260 may include a touch sensor adapted to detect a touch, or a pressure sensor adapted to measure the intensity of force incurred by the touch.

The audio module 1270 may convert a sound into an electrical signal and vice versa. According to an embodiment, the audio module 1270 may obtain the sound via the input module 1250, or output the sound via the sound output module #55 or a headphone of an external electronic device (e.g., an electronic device #02) directly (e.g., wiredly) or wirelessly coupled with the electronic device 1201.

The sensor module 1276 may detect an operational state (e.g., power or temperature) of the electronic device 1201 or an environmental state (e.g., a state of a user) external to the electronic device 1201, and then generate an electrical signal or data value corresponding to the detected state. According to an embodiment, the sensor module 1276 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 1277 may support one or more specified protocols to be used for the electronic device 1201 to be coupled with the external electronic device (e.g., the electronic device 1202) directly (e.g., wiredly) or wirelessly. According to an embodiment, the interface 1277 may include, for example, a high definition multimedia interface (HDMI), a universal serial bus (USB) interface, a secure digital (SD) card interface, or an audio interface.

A connecting terminal 1278 may include a connector via which the electronic device 1201 may be physically connected with the external electronic device (e.g., the electronic device 1202). According to an embodiment, the connecting terminal 1278 may include, for example, a HDMI connector, a USB connector, a SD card connector, or an audio connector (e.g., a headphone connector).

The haptic module 1279 may convert an electrical signal into a mechanical stimulus (e.g., a vibration or a movement) or electrical stimulus which may be recognized by a user via his tactile sensation or kinesthetic sensation. According to an embodiment, the haptic module 1279 may include, for example, a motor, a piezoelectric element, or an electric stimulator.

The camera module 1280 may capture a still image or moving images. According to an embodiment, the camera module 1280 may include one or more lenses, image sensors, image signal processors, or flashes.

The power management module 1288 may manage power supplied to the electronic device 1201. According to one embodiment, the power management module 1288 may be implemented as at least part of, for example, a power management integrated circuit (PMIC).

The battery 1289 may supply power to at least one component of the electronic device #01. According to an embodiment, the battery 1289 may include, for example, a primary cell which is not rechargeable, a secondary cell which is rechargeable, or a fuel cell.

The communication module 1290 may support establishing a direct (e.g., wired) communication channel or a wireless communication channel between the electronic device 1201 and the external electronic device (e.g., the electronic device 1202, the electronic device 1204, or the server 1208) and performing communication via the established communication channel. The communication module 1290 may include one or more communication processors that are operable independently from the processor 1220 (e.g., the application processor (AP)) and supports a direct (e.g., wired) communication or a wireless communication. According to an embodiment, the communication module 1290 may include a wireless communication module 1292 (e.g., a cellular communication module, a short-range wireless communication module, or a global navigation satellite system (GNSS) communication module) or a wired communication module 1294 (e.g., a local area network (LAN) communication module or a power line communication (PLC) module). A corresponding one of these communication modules may communicate with the external electronic device via the first network 1298 (e.g., a short-range communication network, such as Bluetooth™, wireless-fidelity (Wi-Fi) direct, or infrared data association (IrDA)) or the second network 1299 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 1292 may identify and authenticate the electronic device 1201 in a communication network, such as the first network 1298 or the second network 1299, using subscriber information (e.g., international mobile subscriber identity (IMSI)) stored in the subscriber identification module 1296.

The wireless communication module 1292 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 1292 may support a high-frequency band (e.g., the mmWave band) to achieve, e.g., a high data transmission rate. The wireless communication module 1292 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 1292 may support various requirements specified in the electronic device 1201, an external electronic device (e.g., the electronic device 1204), or a network system (e.g., the second network 1299). According to an embodiment, the wireless communication module 1292 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 1297 may transmit or receive a signal or power to or from the outside (e.g., the external electronic device) of the electronic device 1201. According to an embodiment, the antenna module 1297 may include an antenna including a radiating element composed of a conductive material or a conductive pattern formed in or on a substrate (e.g., a printed circuit board (PCB)). According to an embodiment, the antenna module 1297 may include a plurality of antennas (e.g., array antennas). In such a case, at least one antenna appropriate for a communication scheme used in the communication network, such as the first network 1298 or the second network 1299, may be selected, for example, by the communication module 1290 (e.g., the wireless communication module 1292) from the plurality of antennas. The signal or the power may then be transmitted or received between the communication module 1290 and the external electronic device via the selected at least one antenna. According to an embodiment, another component (e.g., a radio frequency integrated circuit (RFIC)) other than the radiating element may be additionally formed as part of the antenna module 1297.

According to various embodiments, the antenna module 1297 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adj acent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 1201 and the external electronic device 1204 via the server 1208 coupled with the second network 1299. Each of the electronic devices 1202 or 1204 may be a device of a same type as, or a different type, from the electronic device 1201. According to an embodiment, all or some of operations to be executed at the electronic device 1201 may be executed at one or more of the external electronic devices 1202, 1204, or 1208. For example, if the electronic device 1201 should perform a function or a service automatically, or in response to a request from a user or another device, the electronic device 1201, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 1201. The electronic device 1201 may provide the outcome, with or without further processing of the outcome, as at least part of a reply to the request. To that end, a cloud computing, distributed computing, mobile edge computing (MEC), or client-server computing technology may be used, for example. The electronic device 1201 may provide ultra low-latency services using, e.g., distributed computing or mobile edge computing. In another embodiment, the external electronic device 1204 may include an internet-of-things (IoT) device. The server 1208 may be an intelligent server using machine learning and/or a neural network. According to an embodiment, the external electronic device 1204 or the server 1208 may be included in the second network 1299. The electronic device 1201 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 1240) including one or more instructions that are stored in a storage medium (e.g., internal memory 1236 or external memory 1238) that is readable by a machine (e.g., the electronic device 1201). For example, a processor (e.g., the processor 1220) of the machine (e.g., the electronic device 1201) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

**Claims**

1. A wearable device comprising:

   A housing comprising a first surface configured to come into contact with a first portion of a human body of a user when worn, a second surface configured to not come into contact with the first portion of the human body in case that the first surface comes into contact with the first portion, and a lateral surface surrounding at least a portion of a space between the first surface and the second surface;
   a biometric sensor disposed inside the housing and comprising a first electrode and a second electrode exposed through the first surface and a third electrode and a fourth electrode exposed through at least one of the second surface and the lateral surface;
   a voltage measurement part configured to measure a voltage at both ends of the first electrode and the fourth electrode caused by a current applied to an object through the second electrode and the third electrode; and
   a processor electrically connected to the biometric sensor,
   wherein the processor is configured to:

   detect, through the biometric sensor, that the first portion of the human body comes into contact with the first electrode and the second electrode;
   detect, through the biometric sensor, that a second portion of the human body comes into contact with the third electrode and the fourth electrode;
   acquire impedance phase information of the human body based on a current applied to an object through the second electrode and the third electrode and a voltage at the both ends of the first electrode and the fourth electrode;
   determine whether the acquired impedance phase information falls within a predetermined range; and
   in case that the acquired impedance information deviates from a predetermined range, provide a notification related to biometric data measurement.

2. The wearable device of claim 1, wherein at least one of the third electrode and the fourth electrode is disposed on the lateral surface of the housing.

3. The wearable device of claim 1, wherein at least one of the third electrode and the fourth electrode is disposed on an upper surface of the housing.

4. The wearable device of claim 1, further comprising a display,
   wherein the notification comprises a guide for a measurement method provided through the display.

5. The wearable device of claim 1, further comprising a motor,
   wherein the notification comprises vibration generated by using the motor.

6. The wearable device of claim 1, further comprising a speaker,
wherein the notification comprises a voice guide or sound output through the speaker.

7. The wearable device of claim 1, further comprising a memory,
wherein in case that the processor receives at least one piece of information on a user's height, weight, age, and gender, the memory is configured to store the at least one piece of information.

8. The wearable device of claim 1, wherein in case that the acquired impedance phase information falls within a first range deviating from the predetermined range, the processor is configured to provide a notification of a measurement error.

9. The wearable device of claim 8, wherein in case that the acquired impedance phase information deviates from the predetermined range and falls within a second range different from the first range, the processor is configured to provide a notification for an electrode error.

10. The wearable device of claim 1, wherein the biometric sensor comprises at least one of an electrocardiogram (ECG) sensor and a bioelectrical impedance analysis (BIA) sensor, and
wherein the processor is configured to store the biometric data acquired from the biometric sensor in a memory.

11. The wearable device of claim 1, wherein the processor is configured to:

detect, through the biometric sensor, that the first portion of the human body comes into contact with the first electrode and the second electrode; and
in case of detecting that the second portion of the human body comes into contact with the third electrode and the fourth electrode through the biometric sensor, automatically start biometric data measurement.

12. The wearable device of claim 1, wherein the processor is configured to:

detect a measurement mode;
detect, through the biometric sensor, that the first portion of the human body comes into contact with the first electrode and the second electrode; and
in case of detecting that the second portion of the human body comes into contact with the third electrode and the fourth electrode through the biometric sensor, start biometric data measurement.

13. The wearable device of claim 1,

wherein in a first section, whether the acquired phase information falls within a predetermined range is determined based on first accuracy,
wherein in a second section, whether the acquired phase information falls within a predetermined range is determined based on second accuracy, and
wherein the first accuracy and the second accuracy vary based on the biometric information acquired from the user.

14. The wearable device of claim 13, wherein in case of acquiring the impedance phase information based on a current applied to an object through the second electrode and the third electrode and a voltage at the both ends of the first electrode and the fourth electrode,

wherein the impedance phase information is acquired during a first time in the first section,
wherein the impedance phase information is acquired during a second time in the second section, and
the second time varies based on the first accuracy.

15. An operating method of a wearable device, the operating method comprising:

detecting, through a biometric sensor, that a first portion of a user human body comes into contact with a first electrode and a second electrode;
detecting, through the biometric sensor, that a second portion of the user human body comes into contact with a third electrode and a fourth electrode;
acquiring impedance phase information of the human body by using the first electrode, the second electrode, the third electrode, and the fourth electrode;
determining whether the acquired impedance phase information falls within a predetermined range; and
in case that the acquired impedance information deviates from the predetermined range, providing a notification related to biometric data measurement.

101

100

FIG.1

# FIG.2A

121

111

100

101

120

150

160

130

170

180

130

190

193

140

142

146

FIG.2B

146

142

140

193

FIG.2C

100

Display (320) ⟷ Processor (310) ⟷ Speaker (340)

Memory (330) ⟷ Motor (350)

⟷ LED (360)

Sensor part (370)

First electrode (370a)  Second electrode (370b)  Third electrode (370c)  Fourth electrode (370d)

First area (380)  Second area (390)

FIG.3

370b

370c

370d

Body Impedance
Measuring...

370a

FIG.4

FIG.5

FIG.6A

FIG.6B

FIG.6C

```
                    ┌─────────────┐
                    │    START    │
                    └──────┬──────┘
                           │
                           ▼
        ┌──────────────────────────────────────┐
        │  DETECT THAT FIRST PORTION OF HUMAN   │
        │  BODY COMES INTO CONTACT WITH FIRST   │ ∼ 710
        │   ELECTRODE AND SECOND ELECTRODE      │
        └──────────────────┬───────────────────┘
                           │
                           ▼
        ┌──────────────────────────────────────┐
        │  DETECT THAT SECOND PORTION OF HUMAN  │
        │  BODY COMES INTO CONTACT WITH THIRD   │ ∼ 720
        │    ELECTRODE AND FOURTH ELECTRODE     │
        └──────────────────┬───────────────────┘
                           │
                           ▼
        ┌──────────────────────────────────────┐
        │  ACQUIRE IMPEDANCE PHASE INFORMATION  │ ∼ 730
        │            OF HUMAN BODY              │
        └──────────────────┬───────────────────┘
                           │
                           ▼
        ┌──────────────────────────────────────┐
        │     DETERMINE WHETHER ACQUIRED        │
        │      IMPEDANCE PHASE INFORMATION      │ ∼ 740
        │   FALLS WITHIN PREDETERMINED RANGE    │
        └──────────────────┬───────────────────┘
                           │
                           ▼
        ┌──────────────────────────────────────┐
        │     PROVIDE NOTIFICATION IN CASE THAT │
        │  ACQUIRED PHASE INFORMATION DEVIATES  │ ∼ 750
        │       FROM PREDETERMINED RANGE        │
        └──────────────────┬───────────────────┘
                           │
                           ▼
                    ┌─────────────┐
                    │     END     │
                    └─────────────┘
```

# FIG.7

INPUT INFORMATION ON USER'S HEIGHT, WEIGHT, AGE, AND GENDER ~ 801

GUIDE CORRECT MEASUREMENT POSTURE (ELECTRODE TOUCH LOCATION OF FINGER, WIDENING ARMPITS AS WIDE AS POSSIBLE, AND LEFT HAND AND RIGHT HAND OF USER NOT INTO CONTACT WITH EACH OTHER) ~ 803

START MEASUREMENT ~ 805

ACQUIRE DATA FOR 5-9 SECONDS ~ 807

809
R VALUE NORMAL RANGE DATA IS 75% OR MORE?

NO → INDUCE REMEASUREMENT IN CASE THAT RESISTANCE COMPONENT MEASURED IN HUMAN BODY IS OUT OF RANGE OR VARIATION IS LARGE FOR 4 SECONDS 813

YES ↓

811
PHASE VALUE NORMAL RANGE DATA IS 75% OR MORE?

NO → INDUCE REMEASUREMENT SINCE PHASE VALUE EXCEEDING THRESHOLD OCCURS IN CASE THAT LEFT HAND AND RIGHT HAND OF USER COME INTO CONTACT WITH OTHER AREAS EXCLUDING ELECTRODE 817

YES ↓

PROCEED ADDITIONAL MEASUREMENT FOR 3 SECONDS ~ 815

819
R AND PHASE VALUE NORMAL RANGE DATA IS 50% OR MORE?

NO → INDUCE REMEASUREMENT IN CASE THAT, AFTER JUDGEMENT AS NORMAL POSTURE BASED ON DATA OF INITIAL 5 TO 9 SECONDS, THERE IS ERROR DATA OF 50% OR MORE DUE TO SHAKING 823

YES ↓

FILTER OUT ABNORMAL VALUES, FOLLOWED BY AVERAGING AND TRANSFERRING TO BODY COMPONENT ALGORITHM ~ 821

FIG.8

Put your hand on the
BIA electrode

900a

FIG.9A

Body Impedance
Measuring...

900b

# FIG.9B

The BIA recording could not be analyzed because of Bad Signal. If you get this result repeatedly, check the instruction for use.

900c

FIG.9C

Body Composition Report

25     Male     175cm
Age     Eender     Height

MEASUREMENTS

| Body Weight | Body Fat | BMI |
|---|---|---|
| 72kg | 8% | 24.8 kg/m² |

BODY FAT ANALYSIS

5kg
Total Body
Fat

900d

# FIG.9D

FIG.9E

FIG.10

FIG.11A

1100

Capacitance
up

AC
Source ~1110

1130~ Z_body  Z_contect ~1140

DC
detector ~1120

# FIG.11B

FIG.11C

FIG.12

**EP 4 166 075 A1**

## INTERNATIONAL SEARCH REPORT

| International application No. |
|---|
| **PCT/KR2021/009122** |

### A. CLASSIFICATION OF SUBJECT MATTER

**A61B 5/053**(2006.01)i; **A61B 5/00**(2006.01)i

According to International Patent Classification (IPC) or to both national classification and IPC

### B. FIELDS SEARCHED

Minimum documentation searched (classification system followed by classification symbols)

A61B 5/053(2006.01); A61B 5/00(2006.01); A61B 5/01(2006.01); A61B 5/024(2006.01); G06F 1/32(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 웨어러블 (wearable), 접촉 (contact), 센서 (sensor), 전극 (electrode), 전압 (voltage), 임피던스 (impedance)

### C. DOCUMENTS CONSIDERED TO BE RELEVANT

| Category* | Citation of document, with indication, where appropriate, of the relevant passages | Relevant to claim No. |
|---|---|---|
| Y | KR 10-2096804 B1 (INBODY CO., LTD.) 03 April 2020 (2020-04-03)<br>See paragraphs [0036]-[0066]; claims 1, 11, 12 and 14; and figures 3a-4b. | 1-15 |
| Y | KR 10-2017-0072700 A (SAMSUNG ELECTRONICS CO., LTD.) 27 June 2017 (2017-06-27)<br>See paragraphs [0066] and [0067]. | 1-15 |
| A | KR 10-2016-0141567 A (DASAN KYS.INC) 09 December 2016 (2016-12-09)<br>See entire document. | 1-15 |
| A | KR 10-2019-0097474 A (SAMSUNG ELECTRONICS CO., LTD.) 21 August 2019 (2019-08-21)<br>See entire document. | 1-15 |
| A | KR 10-2018-0096295 A (SAMSUNG ELECTRONICS CO., LTD.) 29 August 2018 (2018-08-29)<br>See entire document. | 1-15 |

☐ Further documents are listed in the continuation of Box C.      ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **05 November 2021** | **05 November 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

# EP 4 166 075 A1

**INTERNATIONAL SEARCH REPORT**
Information on patent family members

International application No.

**PCT/KR2021/009122**

| Patent document cited in search report | | | Publication date (day/month/year) | Patent family member(s) | | | Publication date (day/month/year) |
|---|---|---|---|---|---|---|---|
| KR | 10-2096804 | B1 | 03 April 2020 | CN | 110114741 | A | 09 August 2019 |
| | | | | KR | 10-2018-0078164 | A | 09 July 2018 |
| | | | | US | 2020-0064906 | A1 | 27 February 2020 |
| | | | | WO | 2018-124809 | A1 | 05 July 2018 |
| KR | 10-2017-0072700 | A | 27 June 2017 | US | 10420483 | B2 | 24 September 2019 |
| | | | | US | 2017-0172452 | A1 | 22 June 2017 |
| KR | 10-2016-0141567 | A | 09 December 2016 | KR | 10-1692004 | B1 | 03 January 2017 |
| KR | 10-2019-0097474 | A | 21 August 2019 | US | 2021-0000376 | A1 | 07 January 2021 |
| | | | | WO | 2019-156320 | A1 | 15 August 2019 |
| KR | 10-2018-0096295 | A | 29 August 2018 | US | 2018-0235542 | A1 | 23 August 2018 |